(19) Europäisches Patentamt
European Patent Office
Office européen des brevets

(11) EP 1 590 007 B1

(12) **EUROPEAN PATENT SPECIFICATION**

(45) Date of publication and mention of the grant of the patent:
**10.11.2010 Bulletin 2010/45**

(21) Application number: **04709267.1**

(22) Date of filing: **09.02.2004**

(51) Int Cl.:
*A61K 51/04 (2006.01)* *A61K 51/12 (2006.01)*
*A61K 49/00 (2006.01)* *C07D 451/02 (2006.01)*

(86) International application number:
**PCT/GB2004/000443**

(87) International publication number:
**WO 2004/069285 (19.08.2004 Gazette 2004/34)**

(54) **IMPROVED RADIOMETAL COMPLEX COMPOSITIONS**

VERBESSERTE RADIOMETALL-KOMPLEX-ZUSAMMENSETZUNGEN

COMPOSITIONS AMELIOREES DE COMPLEXE DE RADIOMETAL

(84) Designated Contracting States:
**AT BE BG CH CY CZ DE DK EE ES FI FR GB GR HU IE IT LI LU MC NL PT RO SE SI SK TR**

(30) Priority: **07.02.2003 EP 03002809**

(43) Date of publication of application:
**02.11.2005 Bulletin 2005/44**

(73) Proprietor: **GE Healthcare Limited**
**Little Chalfont**
**Buckinghamshire HP7 9NA (GB)**

(72) Inventors:
- **MCGILL, David**
  **Amersham plc**
  **Amersham**
  **Buckinghamshire HP7 9LL (GB)**
- **HENRIKSEN, Ingrid**
  **Postboks 4220 Nydalen**
  **0401 Oslo (NO)**

(74) Representative: **Canning, Lewis R. et al**
**GE Healthcare Limited**
**Amersham Place**
**Little Chalfont**
**Buckinghamshire HP7 9NA (GB)**

(56) References cited:
**EP-A- 0 004 684** **EP-A- 0 078 642**
**WO-A-01/40239** **WO-A-97/16210**
**WO-A-98/55154** **WO-A-02/053192**
**GB-A- 1 489 330** **US-A- 5 980 860**

- **KUNG H F: "Development of Tc-99m labeled tropanes: TRODAT-1, as a dopamine transporter imaging agent" NUCLEAR MEDICINE AND BIOLOGY, ELSEVIER SCIENCE PUBLISHERS, NEW YORK, NY, US, vol. 28, no. 5, July 2001 (2001-07), pages 505-508, XP004247058 ISSN: 0969-8051 cited in the application**
- **MELTZER PC ET AL: "A technetium-99m SPECT imaging agent which targets the dopamine transporter in primate brain" JOURNAL OF MEDICINAL CHEMISTRY, AMERICAN CHEMICAL SOCIETY. WASHINGTON, US, vol. 40, no. 12, 1997, pages 1835-1844, XP002194127 ISSN: 0022-2623 cited in the application**
- **MEEGALLA ET AL: "Synthesis and Characterization of Technetium-99m-Labeled Tropanes as Dopamine Transporter-Imaging Agents" JOURNAL OF MEDICINAL CHEMISTRY, AMERICAN CHEMICAL SOCIETY. WASHINGTON, US, vol. 40, no. 1, 1997, pages 9-17, XP002194129 ISSN: 0022-2623 cited in the application**
- **FANG P ET AL: "Preparation and animal studies of [Tc-99m]-TRODAT-1 as a dopamine transporter imaging agent" CHINESE JOURNAL OF NUCLEAR MEDICINE, vol. 19, no. 3, August 1999 (1999-08), pages 146-8, XP009013496 cited in the application**
- **CHOI S R ET AL: "An improved kit formulation of a dopamine transporter imaging agent: [Tc-99m] TRODAT-1 - Transchelation preparation of Tc-99m-sestamibi" NUCLEAR MEDICINE AND BIOLOGY, ELSEVIER SCIENCE PUBLISHERS, NEW YORK, NY, US, vol. 26, no. 4, May 1999 (1999-05), pages 461-466, XP004167081 ISSN: 0969-8051 cited in the application**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 590 007 B1

- JURISSON S S ET AL: "Potential technetium small molecule radiopharmaceuticals" CHEMICAL REVIEWS, vol. 99, no. 9, September 1999 (1999-09), pages 2205-2218, XP000852431 ISSN: 0009-2665 cited in the application
- TURPIN F ET AL: "Synthesis of two novel oxocyclam-binding technetium complexes containing an analogue of cocaine." JOURNAL OF LABELLED COMPOUNDS AND RADIOPHARMACEUTICALS, vol. 45, no. 5, April 2002 (2002-04), pages 379-393, XP002256764 April, 2002 ISSN: 0362-4803 cited in the application
- ARANO YASUSHI: "Delivery of diagnostic agents for gamma-imaging" ADVANCED DRUG DELIVERY REVIEWS, vol. 37, no. 1, 4 May 1999 (1999-05-04), pages 103-120, XP002256765 cited in the application
- MEEGALLA SANATH K ET AL: "Specificity of diastereomers of (99mTc)TRODAT-1 as dopamine transporter imaging agents." JOURNAL OF MEDICINAL CHEMISTRY, vol. 41, no. 4, 12 February 1998 (1998-02-12), pages 428-436, XP002256766 ISSN: 0022-2623 cited in the application

## Description

Field of the Invention.

The present invention relates to stabilised technetium and rhenium metal complex compositions comprising a radioprotectant and a radiometal complex of a tropane-tetradentate chelating agent conjugate. Radiopharmaceuticals comprising the stabilised metal complex compositions, and kits for the preparation of the radiopharmaceuticals are also described.

Background to the Invention.

Tropanes labelled with $^{123}I$, $^{18}F$ or $^{99m}Tc$ are known as diagnostic imaging radiopharmaceuticals for brain imaging [Morgan and Nowotnik, Drug News Perspect. 12(3), 137-145 (1999)]. Tropanes are known to target the dopamine transporter in the brain, and the dopamine transporter has been implicated in several diseases including Parkinson's Disease, Parkinsonian Syndrome and attention-deficit hyperactivity disorder.

Tropanes labelled with $^{99m}Tc$ are known. The development of $^{99m}Tc$-TRODAT-1 has been described by Kung [Nucl.Med.Biol., 28, p.505-508 (2001)]:

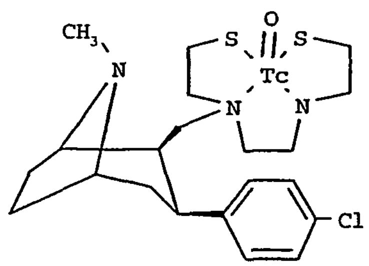

$^{99m}$Tc-TRODAT-1

TRODAT-1 is also described in US 5980860 and equivalents.

Technepine has also been described by Meltzer et al [J.Med.Chem., 40, 1835-1844 (1997)]:

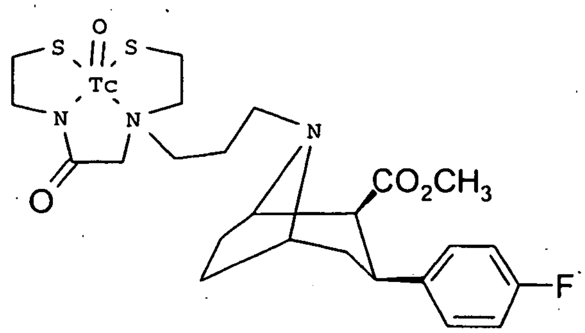

Technepine

Technepine is described in US 6171576 and equivalents.

WO 03/055879 describes chelator-tropane conjugates wherein the 6- or 7- positions of the tropane are functionalised. Kits are briefly described, but the use of radioprotectants is not disclosed.

A range of $^{99m}Tc$ complexes of $N_2S_2$ diaminedithiol chelator conjugates of tropanes (including TROD AT-1) have been reported to show good *in vitro* stability at 4 and 24 hours post preparation, with little change in radiochemical purity [Meegalla et al, J.Med.Chem., 40, p.9-17 (1997)]. Fan et al [Chin.J.Nucl.Med., 19(3) 146-148 (1999)] report that $^{99m}Tc$-TRODAT-1 is stable for 24 hours at room temperature.

An improved kit formulation for the preparation of $^{99m}$Tc-TRODAT-1 has been described [Choi et al, Nucl.Med.Biol., 26, p. 461-466 (1999)]. Choi *et al* report that, as long as a minimum of 10 μg (microgrammes) of the tropane conjugate is present in the kit, the radiochemical purity consistently reaches greater than 90%. Heating is necessary to achieve a satisfactory radiochemical purity (RCP), and Choi *et al* use autoclave heating for 30 minutes.

WO 02/053192 discloses stabilised $^{99m}$Tc metal complex compositions comprising a radioprotectant (chosen from ascorbic acid, *para*-aminobenzoic acid, gentisic acid or biocompatible salts thereof), together with an antimicrobial preservative of the paraben type.

WO 98/55154 discloses a method of inhibiting the degradation of radiolabelled peptides using povidone, optionally with secondary stabilisers chosen from ascorbic acid, benzyl alcohol, cysteamine, cystamine, propylene glycol, dextran and gentisic acid.

EP 0078642 A discloses stabilised $^{99m}$Tc compositions, having a stabilizing agent, where the stabilizing agent is of formula:

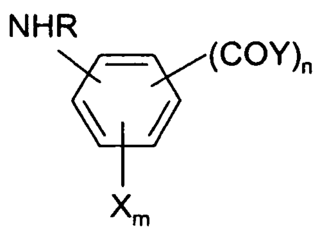

where:

R is $C_{1-6}$ alkyl or H;
X is $C_{1-6}$ alkyl or OH;
m is 0, 1 or 2;
Y is OH or $-NHCH_2COOH$;
n is 1 or 2;

or a salt ester or amide thereof.

EP 0004684 discloses stabilised compositions useful in the preparation of $^{99m}$Tc scanning agents, where the stabiliser is gentisic acid or pharmaceutically acceptable salts or esters thereof.

GB 1489330 discloses stabilised $^{99m}$Tc compositions, wherein the stabiliser is ascorbic acid, erythrorbic acid, or pharmaceutically acceptable inorganic salts thereof.

<u>The Present Invention.</u>

Technetium-99m ($^{99m}$Tc) is a radioisotope which decays with a half-life of 6.02 hours to technetium-99 ($^{99}$Tc). The radioactive decay is accompanied by the emission of a gamma ray with an energy that is near ideal for medical imaging with a modem gamma-camera. The decay product, $^{99}$Tc, is also radioactive and decays by β-emission with a half-life of $2.1\times10^5$ years (to the stable isotope $^{99}$Ru), but the radioactive emissions from $^{99}$Tc are insufficient for medical imaging. Conventional $^{99m}$Tc "generators" comprise the radioisotope $^{99}$Mo, which decays with a half-life of 66.2 hours. About 86% of $^{99}$Mo decays result in the production of $^{99m}$Tc, however ca. 14% of $^{99}$Mo decays result in the direct production of $^{99}$Tc. Therefore, if a $^{99m}$Tc generator is eluted a very short time after the previous elution, the $^{99m}$Tc content will be low but will be about 86% of the total technetium content. As time passes since the previous elution of the generator, $^{99}$Tc is being produced both from $^{99}$Mo and from the decay of $^{99m}$Tc to $^{99}$Tc. Consequently, as the time interval between generator elutions increases, the $^{99}$Tc/$^{99m}$Tc ratio increases. The $^{99}$Tc and $^{99m}$Tc technetium isotopes are chemically identical, and consequently any radiopharmaceutical preparation must be able to cope with a wide range of $^{99}$Tc chemical content in the eluate in order to be able to function effectively over the usable lifetime of the generator. It is also the case that elutions made with a fresh $^{99m}$Tc generator are likely to have a higher radioactive concentration, and thus have a higher concentration of reactive free radicals arising from radiolysis of the solvent (water). A viable $^{99m}$Tc radiopharmaceutical preparation thus needs to be able to provide satisfactory RCP performance even when such reactive free radicals are present. These characteristics of the $^{99m}$Tc generator are illustrated in most radiochemistry or nuclear chemistry textbooks, and the problems that different eluate properties can give to the performance of $^{99m}$Tc kits have been described by Saha, G. B. "Radiopharmaceuticals and Methods of Radiolabeling"; Chapter 6 (pages 80-108) in Fundamentals of Nuclear Pharmacy (3rd Edn.), and Hung et al for Cardiolite™ [Nucl. Med. Biol., 23, 599 - 603 (1996)].

The present invention provides improved radiometal complex compositions comprising a technetium or rhenium metal complex of a tropane-tetradentate chelating agent conjugate and a radioprotectant. The improved compositions exhibit more reproducible initial radiochemical purity (RCP) and improved stability post-reconstitution, so that an RCP of 85 to 90% is maintained at 6 hours post-reconstitution. The problem of unsatisfactory RCP for radiometal tropane conjugates under certain conditions of radioactivity levels, radioactive concentrations or reconstitution volumes was not recognised in the prior art. Such conditions are those that could arise under normal conditions of use of a commercial radionuclide generator, such as a $^{99m}$Tc generator. The present invention provides compositions comprising a radioprotectant which solve this previously unrecognised problem.

Detailed Description of the Invention.

In a first embodiment, the present invention provides a stabilised composition which comprises:

(i) a metal complex of a radioactive isotope of technetium or rhenium chelated to a conjugate, wherein said conjugate comprises a tetradentate chelating agent conjugated to a tropane, and said tetradentate chelating agent forms a neutral metal complex with said radioactive isotope of technetium or rhenium;
(ii) at least one radioprotectant.

The term "tropane" has its conventional meaning, i.e. a bicyclic amine of formula (with numbering of the ring positions shown):

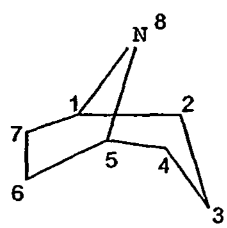

where the amine nitrogen at the 8-position may be secondary or tertiary.

By the term "metal complex" is meant a coordination complex of the technetium or rhenium metal ion with a ligand, here the tetradentate chelating agent. The chelated metal complex is "resistant to transchelation", i.e. does not readily undergo ligand exchange with other potentially competing ligands for the radiometal coordination sites. Potentially competing ligands include the tropane moiety itself, the radioprotectant or other excipients in the preparation *in vitro* (e.g. antimicrobial preservatives), or endogenous compounds *in vivo* (e.g. glutathione, transferrin or plasma proteins).

Suitable radioactive isotopes of technetium or rhenium include: $^{94m}$Tc, $^{99m}$Tc, $^{186}$Re and $^{188}$Re. A preferred such radioisotope is $^{99m}$Tc.

The term "tetradentate" has its conventional meaning, i.e. the chelating agent has four donor atoms, each of which coordinate to the metal giving chelate rings on formation of the metal complex. The tetradentate chelating agent is preferably attached at the 2-, 6-, 7-or 8- positions of the tropane, and is most preferably attached at either the 2- or the 8-position of the tropane, ideally at the 2-position.

By the term "radioprotectant" is meant a compound which inhibits degradation reactions induced by radioactive emissions (e.g. redox processes), by trapping highly-reactive free radicals, such as oxygen-containing free radicals arising from the radiolysis of water. The radioprotectants of the present invention are suitably chosen from: ascorbic acid, *para*-aminobenzoic acid (i.e. 4-aminobenzoic acid), gentisic acid (i.e. 2,5-dihydroxybenzoic acid), gentisyl alcohol and salicyclic acid, including salts thereof with a biocompatible cation. Preferred radioprotectants are ascorbic acid and *para*-aminobenzoic acid, or salts thereof with a biocompatible cation. Especially preferred radioprotectants are ascorbic acid and salts thereof with a biocompatible cation. A preferred such salt is sodium ascorbate. The radioprotectants of the present invention are commercially available to a pharmaceutical grade specification.

By the term "biocompatible cation" is meant a positively charged counterion which forms a salt with an ionised, negatively charged group, where said positively charged counterion is also non-toxic and hence suitable for administration to the mammalian body, especially the human body. Examples of suitable biocompatible cations include: the alkali metals sodium or potassium; the alkaline earth metals calcium and magnesium; and the ammonium ion. Preferred biocompatible cations are sodium and potassium, most preferably sodium.

The technetium and rhenium metal complexes of the present invention are "neutral", i.e. any positive charge on the central metal core is balanced by the sum of the negative charge on the four metal donor atoms of the tetradentate chelating agent, to give an overall electrically neutral metal complex. Examples of likely technetium cores are $O=Tc^{+}=O$

and $Tc^{3+}=O$, which both represent technetium in the Tc(V) oxidation state. Similar cores $O=Re^{+}=O$ and $Re^{3+}=O$ are known for rhenium.

The neutral radioactive technetium or rhenium complexes of the present invention are suitably of Formula I:

$$[\{tropane\}-(A)_n]_m-[metal\ complex] \quad (I)$$

where: $(A)_n$ is a linker group,
n is an integer of value 0 to 10,
and m is 1, 2 or 3.

The "linker group" $(A)_n$ is as defined below for Formula Ia. The metal complexes of Formula I are derived from tropane "conjugates". The tropane tetradentate chelating agent "conjugates" of the present invention are as defined in Formula Ia:

$$[\{tropane\}-(A)_n]_m-[tetradentate\ chelating\ agent] \quad (Ia)$$

where:

$-(A)_n-$ is a linker group wherein each A is independently $-CR_2-$, -CR=CR-, -C≡C- , $-CR_2CO_2-$ , $-CO_2CR_2-$ , NRCO- , -CONR- , NR(C=O)NR-, -NR(C=S)NR-, $-SO_2NR-$ , $-NRSO_2-$ , $-CR_2OCR_2-$ , $-CR_2SCR_2-$ , $-CR_2NRCR_2-$ , a $C_{4-8}$ cycloheteroalkylene group, a $C_{4-8}$ cycloalkylene group, a $C_{5-12}$ arylene group, or a $C_{3-12}$ heteroarylene group;
R is independently chosen from H, $C_{1-4}$ alkyl, $C_{2-4}$ alkenyl, $C_{2-4}$ alkynyl, $C_{1-4}$ alkoxyalkyl or $C_{1-4}$ hydroxyalkyl;
n is an integer of value 0 to 10; and
m is 1, 2 or 3.

In Formulae I and Ia, m is preferably 1 or 2, and is most preferably 1; and $(A)_n$ is preferably $(CR_2)_n$, where n is chosen to be 1 to 3.

Examples of suitable tetradentate chelating agents for technetium and rhenium which form neutral metal complexes include, but are not limited to:

(i) $N_2S_2$ ligands having a diaminedithiol donor set such as BAT, an amideaminedithiol donor set such as MAMA, a phenylenediaminethioetherthiol donor set such as PhAT, or a dithiosemicarbazone donor set;

(ii) diaminedioximes;

(iii) $N_3S$ ligands having a diamidepyridinethiol donor set such as Pica;

(iv) open chain or macrocyclic ligands having an amidetriamine donor set, such as monoxocyclam.

(v) $N_2O_2$ ligands having a diaminediphenol donor set.

The above described ligands are particularly suitable for complexing technetium e.g. $^{94m}Tc$ or $^{99m}Tc$, and are described more fully by Jurisson et al [Chem.Rev., 99, 2205-2218 (1999)]. $N_2S_2$ dithiosemicarbazone chelators are described by Arano et al [Chem.Pharm.Bull., 39, p.104-107. (1991)]. $N_2S_2$ phenylenediaminethioetherthiol chelators are described by McBride et al [J.Med.Chem., 36, p.81-6 (1993)]. Macrocyclic amidetriamine ligands and their tropane conjugates are described by Turpin et al [J.Lab.Comp.Radiopharm., 45, 379-393 (2002)]. Diaminedioximes are described by Nanjappan et al [Tetrahedron, 50, 8617-8632 (1994)]. $N_3S$ ligands having a diamidepyridinethiol donor set such as Pica are described by Bryson et al [Inorg.Chem., 29, 2948-2951 (1990)]. $N_2O_2$ ligands having a diaminediphenol donor set are described by Pillai et al [Appl.Rad.Isot., 41, 557-561 (1990)].

Preferred $^{99m}Tc$ metal complexes of the present invention are those suitable for crossing the blood-brain barrier (BBB) as described by Volkert et al [Radiochim. Acta, 63, p.205-208 (1993)]. Especially preferred $^{99m}Tc$ metal complexes of the present invention are $^{99m}Tc$-TRODAT-1 and Technepine.

Preferred tetradentate chelating agents are those having an $N_2S_2$ diaminedithiol or amideaminedithiol donor set of Formula II:

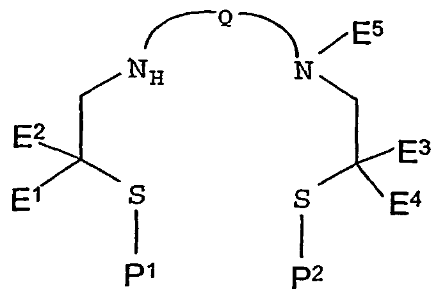

II

where: $E^1$-$E^5$ are each independently an R' group;
each R' is H or $C_{1-10}$ alkyl, $C_{3-10}$ alkylaryl, $C_{2-10}$ alkoxyalkyl, $C_{1-10}$ hydroxyalkyl, $C_{1-10}$ fluoroalkyl, $C_{2-10}$ carboxyalkyl or $C_{1-10}$ aminoalkyl, or two or more R' groups together with the atoms to which they are attached form a carbocyclic, heterocyclic, saturated or unsaturated ring, and wherein one or more of the R' groups is conjugated to the tropane;
and Q is a bridging group of formula $-J(CR'_2)_f-$ ;
where f is 1 or 2, and J is $-CR'_2-$ or C=O;
$P^1$ and $P^2$ are independently H or a thiol protecting group.

By the term "protecting group" is meant a group which inhibits or suppresses undesirable chemical reactions (e.g. oxidation of the free thiol to the corresponding disulphide), but which is designed to be sufficiently reactive that it may be cleaved from the thiol under mild enough conditions that do not modify the rest of the molecule during radiolabelling of the conjugate. Thiol protecting groups are well known to those skilled in the art and include but are not limited to: trityl, 4-methoxybenzyl, benzyl, tetrahydropyranyl, methyltetrahydrofuranyl (MTHF), acetamidomethyl and ethoxyethyl. The use of further thiol protecting groups are described in 'Protective Groups in Organic Synthesis', Theorodora W. Greene and Peter G. M. Wuts, (John Wiley & Sons, 1991). In Formula II, $P^1$ and $P^2$ are preferably both H.

Preferred Q groups are as follows: $-CH_2CH_2-$ , $-CH_2CH_2CH_2-$ or $-(C=O)CH_2-$, most preferably $N_2S_2$ diaminedithiol chelators where Q is $-CH_2CH_2-$ or $-CH_2CH_2CH_2-$, with $-CH_2CH_2-$ (i.e. BAT type chelators) being especially preferred.

$E^1$ to $E^5$ are preferably chosen from: H, $C_{1-3}$ alkyl, $C_{1-3}$ alkoxyalkyl, $C_{1-3}$ hydroxyalkyl or $C_{1-3}$ fluoroalkyl. Most preferably, each $E^1$ to $E^4$ group is H, and $E^5$ is $C_{1-3}$ alkyl.

A most particularly preferred chelator of Formula II is the $N_2S_2$ diaminedithiol chelator of TRODAT-1, i.e. the chelator of Formula II where: Q is $-CH_2CH_2-$ , $E^1$ to $E^5$ are all H and $P^1 = P^2 = H$.

The tetradentate chelating agents of Formula II are preferably attached to the tropane *via* either the bridging group Q or the $E^5$ group. Most preferably, the tropane is attached *via* the $E^5$ group.

Preferably, the tropane of the present invention is a phenyl tropane of Formula III:

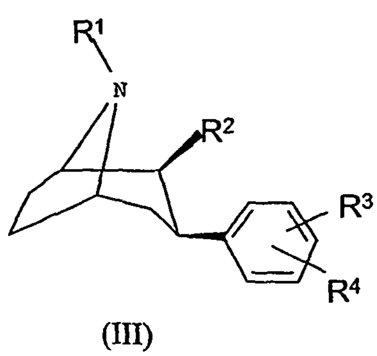

(III)

where:

$R^1$ is H, $C_{1-4}$ alkyl, $C_{1-4}$ alkenyl or $C_{1-4}$ fluoroalkyl;
$R^2$ is $CO_2R^5$, $CON(R^5)_2$, $COR^5$ or $C_{1-4}$ alkyl, where each $R^5$ is independently H or $C_{1-3}$ alkyl;
$R^3$ and $R^4$ are independently H, Cl, Br, F, I, $CH_3$, $C_2H_5$, $CF_3$, $NO_2$, $OCH_3$ or $NH_2$.

$R^1$ is preferably $C_{1-3}$ alkyl or $C_{1-3}$ fluoroalkyl. $R^2$ is preferably $CO_2CH_3$ or $C_{1-2}$ alkyl. $R^3$ is preferably 4-chloro, 4-fluoro or 4-methyl, and $R^4$ is preferably H or $CH_3$. $R^1$ is most preferably $CH_3$.

It is envisaged that the role of the linker group $-(A)_n-$ of Formula I is to distance the relatively bulky metal complex from the tropane, so that binding of the tropane to biological target sites (e.g. the dopamine transporter in the mammalian brain) is not impaired. This can be achieved by a combination of flexibility (e.g. simple alkyl chains), so that the bulky group has the freedom to position itself away from the active site and/or rigidity such as a cycloalkyl or aryl spacer which orientates the metal complex away from the active site.

The nature of the linker group can also be used to modify the biodistribution of the resulting metal complex of the conjugate. Thus, e.g. the introduction of ether groups in the linker will help to minimise plasma protein binding. Preferred linker groups $-(A)_n-$ have a backbone chain of linked atoms which make up the $-(A)_n-$ moiety of 2 to 10 atoms, most preferably 2 to 5 atoms, with 2 or 3 atoms being especially preferred. A minimum linker group backbone chain of 2 atoms confers the advantage that the chelator is well-separated from the tropane, so that any interaction is minimised.

Non-peptide linker groups such as alkylene groups or arylene groups have the advantage that there are no significant hydrogen bonding interactions with the conjugated tropane, so that the linker does not wrap round onto the tropane. Preferred alkylene spacer groups are $-(CH_2)_q-$ where q is 2 to 5. Preferred arylene spacers are of formula:

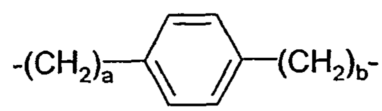

where: a and b are independently 0, 1 or 2.

It is strongly preferred that the tropane is bound to the metal complex in such a way that the linkage does not undergo facile metabolism in blood, since that would result in the metal complex being cleaved off before the labelled tropane inhibitor reached the desired *in vivo* target site. The tropane is therefore preferably covalently bound to the metal complexes of the present invention *via* linkages which are not readily metabolised.

The stabilised composition of the present invention may be prepared by reaction of a solution of the radiometal in the appropriate oxidation state with the chelator conjugate at the appropriate pH in the presence of the radioprotectant, in solution in a suitable solvent. The radioprotectant may be supplied either together with the conjugate or the solution of the radiometal. Preferably, the radioprotectant is pre-mixed with the conjugate, and that precursor composition is subsequently reacted with the radiometal, (as described in the second embodiment below). The conjugate solution may preferably contain a ligand which complexes weakly but rapidly to the radiometal, such as gluconate or citrate i.e. the radiometal complex is prepared by ligand exchange or transchelation. Such conditions are useful to suppress undesirable side reactions such as hydrolysis of the metal ion. When the radiometal is rhenium, the usual radioactive starting material is perrhenate, i.e. $ReO_4^-$. When the radiometal is $^{99m}Tc$, the usual radioactive starting material is sodium pertechnetate from a $^{99}Mo$ generator. In both perrhenate and pertechnetate the metal (M) is present in the M(VII) oxidation state, which is relatively unreactive. The preparation of technetium or rhenium complexes of lower oxidation state M(I) to M(V) therefore usually requires the addition of a suitable biocompatible reductant. The "biocompatible reductant" is a pharmaceutically acceptable reducing agent such as sodium dithionite, sodium bisulphite, formamidine sulphinic acid, stannous ion, Fe(II) or Cu(I), to facilitate complexation. Ascorbic acid can function both as a radioprotectant and a biocompatible reductant, and hence it is possible to use quantities of ascorbic acid greater than that necessary for radioprotection alone to facilitate reduction also. The biocompatible reductant preferably comprises stannous i.e. Sn(II), preferably as a stannous ion or salt. Preferred stannous salts are stannous chloride, stannous fluoride and stannous tartrate. The stannous salt may be employed in either anhydrous or hydrated form.

Alternatively, the stabilised composition of the present invention may be prepared in a stepwise manner by first forming the radiometal complex in a suitable solvent, and subsequently adding the radioprotectant. In such an approach, the radioprotectant should be added as soon as possible after formation of the radiometal complex, so that the stabilising effect of the radioprotectant is brought into effect to minimise radiolysis and possible degradation. Methods of preparation wherein the radioprotectant is present prior to formation of the radiometal complex are preferred.

The concentration of radioprotectant for use in the present invention is suitably 0.3 to 5.0 millimolar, preferably 0.4 to 4.0 millimolar, most preferably 1.0 to 3.5 millimolar. For ascorbic acid, this corresponds to a suitable concentration of 50 to 900 $\mu g/cm^3$, preferably 70 to 800 $\mu g/cm^3$, most preferably 90 to 700 $\mu g/cm^3$. For the $^{99m}Tc$ radiopharmaceutical $^{99m}Tc$-TRODAT-1, the preferred concentration of an ascorbic acid or ascorbate radioprotectant is in the range 0.5 to 3.8 millimolar.

When the radiometal complexes of the present invention are to-be used in radiopharmaceutical compositions, a preferred method of preparation is the use of a sterile, non-radioactive kit as described in the third and fourth embodiments below. The kit provides a convenient supply of the necessary reactants at the right concentration, which needs

only be reconstituted with perrhenate or pertechnetate in saline or another suitable solvent.

In a second embodiment, the present invention provides a precursor composition useful in the preparation of the above stabilised composition, which comprises:

(i) the chelator conjugate of Formula Ia as defined above;
(ii) a radioprotectant as defined above.

Preferably, the tropane of the precursor composition is a phenyl tropane of Formula III (above). Preferred and most preferred phenyl tropanes for the precursor composition are as described above for the first embodiment. Most preferably, the conjugate of the precursor composition is of Formula IV:

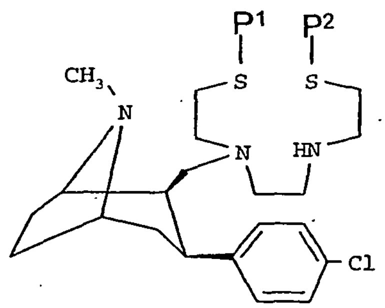

(IV)

where $P^1$ and $P^2$ are independently H or a thiol protecting group.

The term "protecting group" is as defined for Formula II above. Preferred conjugates of Formula IV are where $P^1$ and $P^2$ are both H.

The conjugates used in the precursor compositions of the present invention may be prepared *via* the bifunctional chelate approach. Thus, it is well known to prepare chelating agents which have attached thereto a functional group ("bifunctional chelates"). Functional groups that have been attached include: amine, thiocyanate, maleimide and active esters such as N-hydroxysuccinimide or pentafluorophenol. Such bifunctional chelates can be reacted with suitable functional groups on the tropane to form the desired conjugate. Such suitable functional groups on the tropane include:

carboxyls (for amide bond formation with an amine-functionalised bifunctional chelator);
amines (for amide bond formation with an carboxyl- or active ester-functionalised bifunctional chelator);
halogens, mesylates and tosylates (for N-alkylation of an amine-functionalised bifunctional chelator) and
thiols (for reaction with a maleimide-functionalised bifunctional chelator). Further details of the bifunctional chelate approach are described by Arano [Adv. Drug Deliv. Rev., 37, 103-120 (1999)]. Further details specific to the conjugation of tropanes with the tetradentate chelating agents indicated are described in: the methods of Meegalla et al [J.Med.Chem., 40, 9-17 (1997)] for $N_2S_2$ diaminedithiol chelators; Meltzer et al for N2S2 amideaminedithiol (MAMA) chelators [ibid, 40, 1835-1844 (1997)] and Turpin et al [J.Lab.Comp.Radiopharm., 45, 379-393 (2002)] for monoxocyclam chelators.

In a third embodiment, the present invention provides a radiopharmaceutical which comprises the stabilised composition of the first embodiment together with a biocompatible carrier, in a form suitable for mammalian administration. The "biocompatible carrier" is a fluid, especially a liquid, in which the imaging agent can be suspended or dissolved, such that the composition is physiologically tolerable, i.e. can be administered to the mammalian body without toxicity or undue discomfort. The biocompatible carrier is suitably an injectable carrier liquid such as sterile, pyrogen-free water for injection; an aqueous solution such as saline (which may advantageously be balanced so that the final product for injection is either isotonic or not hypotonic); an aqueous solution of one or more tonicity-adjusting substances (e.g. salts of plasma cations with biocompatible counterions), sugars (e.g. glucose or sucrose), sugar alcohols (e.g. sorbitol or mannitol), glycols (e.g. glycerol), or other non-ionic polyol materials (e.g. polyethyleneglycols, propylene glycols and the like).

The radiopharmaceuticals of the present invention may optionally further comprise an antimicrobial preservative. By the term "antimicrobial preservative" is meant an agent which inhibits the growth of potentially harmful microorganisms such as bacteria, yeasts or moulds. The antimicrobial preservative may also exhibit some bactericidal properties, depending on the concentration. The main role of the antimicrobial preservative(s) of the present invention is to

inhibit the growth of any such micro-organism in the radiopharmaceutical composition post-reconstitution, i.e. in the radioactive diagnostic product itself. Suitable antimicrobial preservative(s) include: the parabens, i.e. methyl, ethyl, propyl or butyl paraben or mixtures thereof; benzyl alcohol; phenol; cresol; cetrimide and thiomersal. Preferred antimicrobial preservative(s) are the parabens.

Such radiopharmaceuticals are suitably supplied in either a container which is provided with a seal which is suitable for single or multiple puncturing with a hypodermic needle (e.g. a crimped-on septum seal closure) whilst maintaining sterile integrity. Such containers may contain single or multiple patient doses. Preferred multiple dose containers comprise a single bulk vial (e.g. of 10 to 30 $cm^3$ volume) which contains multiple patient doses, whereby single patient doses can thus be withdrawn into clinical grade syringes at various time intervals during the viable lifetime of the preparation to suit the clinical situation. Pre-filled syringes are designed to contain a single human dose, and are therefore preferably a disposable or other syringe suitable for clinical use. The pre-filled syringe may optionally be provided with a syringe shield to protect the operator from radioactive dose. Suitable such radiopharmaceutical syringe shields are known in the art and preferably comprise either lead or tungsten.

When the radioactive isotope is $^{99m}Tc$, a radioactivity content suitable for a diagnostic imaging radiopharmaceutical is in the range 180 to 1500 MBq of $^{99m}Tc$, depending on the site to be imaged *in vivo*, the uptake and the target to background ratio. $^{99m}Tc$ is suitable for SPECT imaging and $^{94m}Tc$ for PET imaging.

The radiopharmaceuticals of the present invention comprise the improved radiometal compositions of the first embodiment. This has the advantage that radioactive impurities are suppressed. Such radioactive impurities may either contribute to unnecessary radiation dose for the patient, or may in some cases have an adverse effect on imaging by reducing the signal to background ratio.

The radiopharmaceuticals of the present invention may be prepared from kits, as is described in the fourth embodiment below. Alternatively, the radiometal complexes of the present invention in a biocompatible carrier may be prepared under aseptic manufacture conditions to give the desired sterile product. The radiopharmaceuticals may also be prepared under non-sterile conditions, followed by terminal sterilisation using e.g. gamma-irradiation, autoclaving, dry heat or chemical treatment (e.g. with ethylene oxide). Preferably, the radiopharmaceuticals of the present invention are prepared from kits.

In a fourth embodiment, the present invention provides a kit for the preparation of the radiopharmaceuticals of the present invention, which comprises:

(i) the conjugate of Formula (Ia) or a salt thereof with a biocompatible counterion;
(ii) a radioprotectant (as defined above);
(iii) a biocompatible reductant (as defined above).

Such kits are designed to give sterile radiopharmaceutical products suitable for human administration, e.g. *via* direct injection into the bloodstream. For $^{99m}Tc$, the kit is preferably lyophilised and is designed to be reconstituted with sterile $^{99m}Tc$-pertechnetate ($TcO_4^-$) from a $^{99m}Tc$ radioisotope generator to give a solution suitable for human or mammalian administration without further manipulation. Suitable kits comprise a container (e.g. a septum-sealed vial) containing the conjugate (Ia) in either free base or acid salt form, together with a biocompatible reductant. The "biocompatible reductant" is defined in the first embodiment (above). The biocompatible reductant for the kit is preferably a stannous salt such as stannous chloride or stannous tartrate.

The "radioprotectant" of the kit is as defined above. Preferred radioprotectants correspond to those described for the stabilised composition of the first embodiment.

The conjugate of Formula (Ia) comprises the amine which forms the 8-position of the tropane ring, plus possibly further amine donor atoms of the tetradentate chelating agent. Hence, the conjugate may optionally be used in the kit as "a salt thereof with a biocompatible counterion", i.e. an acid salt of the conjugate. Suitable such salts include but are not limited to: hydrochlorides, trifluoroacetates, sulphonates, tartrates, oxalates and sulphosalicyclates. When the conjugate is of Formula IV, preferred salts are the trifluoroacetate or hydrochloride salts, especially the trifluoroacetate salt.

The non-radioactive kits may optionally further comprise additional components such as one or more transchelator(s), antimicrobial preservative(s), pH-adjusting agent(s) or filler(s). The "transchelator" comprise one or more compounds which react rapidly to form a weak complex(es) with technetium, then are displaced by the ligand. This minimises the risk of formation of reduced hydrolysed technetium (RHT) due to rapid reduction of pertechnetate competing with technetium complexation. Suitable such transchelators are salts of a weak organic acid, i.e. an organic acid having a pKa in the range 3 to 7, with a biocompatible cation. Suitable such weak organic acids are acetic acid, citric acid, tartaric acid, gluconic acid, glucoheptonic acid, benzoic acid, phenols or phosphonic acids, or aminocarboxylic acids, such as ethylenediaminetetraacetic acid (EDTA), iminodiacetic acid (IDA) and nitrilotriacetic acid (NTA). Hence, suitable salts are acetates, citrates, tartrates, gluconates, glucoheptonates, benzoates, phenolates, phosphonates or edetates. Preferred such salts are edetates, gluconates, glucoheptonates, benzoates, or phosphonates, most preferably edetates, gluconates, glucoheptonates or phosphonates, most especially gluconates, glucoheptonates or edetates. Preferred

edetate salts are disodium edetate and calcium edetate. A preferred transchelator is a gluconate or glucoheptonate salt of a biocompatible cation.

When the kit comprises a diaminedithiol $N_2S_2$ tetradentate chelator, the transchelator preferably comprises a combination of a gluconate or glucoheptonate salt, together with an edetate salt.

The "antimicrobial preservative" is as defined for the radiopharmaceutical (i.e. third) embodiment (above). For the kit, the inclusion of an antimicrobial preservative means that, once reconstituted, the growth of potentially harmful micro-organisms in the preparation is inhibited.

The term "pH-adjusting agent" means a compound or mixture of compounds useful to ensure that the pH of the reconstituted kit is within acceptable limits (approximately pH 4.0 to 10.5) for human or mammalian administration. Suitable such pH-adjusting agents include pharmaceutically acceptable buffers, such as tricine, phosphate or TRIS [i.e. *tris*(hydroxymethyl)aminomethane], and pharmaceutically acceptable bases such as sodium carbonate, sodium bicarbonate or mixtures thereof. When the conjugate is employed in acid salt form, the pH adjusting agent may optionally be provided in a separate vial or container, so that the user of the kit can adjust the pH as part of a multi-step procedure.

By the term "filler" is meant a pharmaceutically acceptable bulking agent which may facilitate material handling during production and lyophilisation. Suitable fillers include inorganic salts such as sodium chloride, and water soluble sugars or sugar alcohols such as sucrose, maltose, mannitol or trehalose. A preferred filler is mannitol.

For $^{99m}Tc$, the kit is preferably lyophilised and is designed to be reconstituted with a sterile solution of $^{99m}Tc$-pertechnetate ($TcO_4^-$) from a $^{99m}Tc$ radioisotope generator to give a solution suitable for human or mammalian administration with the minimum of further manipulation. In ideal circumstances, the desired radiopharmaceutical product is formed at room temperature in a few minutes directly from $^{99m}Tc$ generator eluate, i.e. a one-step preparation. An alternative possibility is a multi-step process in which it is necessary to add two or more solutions (e.g. eluate and buffer solution) to the kit. In some instances, the reaction time at room temperature may be found to be unduly long. This can readily be determined by RCP measurements at time intervals as is known in the art. Heating may therefore need to be applied to drive the radiolabelling reaction to completion in a shorter timeframe. When heating is necessary, the heating process may employ any suitable methodology such as: hot baths of fluid, such as water or a high-boiling oil (e.g. silicone); heating blocks; hot plates or microwave radiation; as long as the desired temperature control can be achieved. After the heating is complete, the reaction mixture is either allowed to cool to room temperature, or may be actively cooled (e.g. in a stream of a cooling fluid such as a gas or water) or *via* a heating block with integral inductive cooling.

Preferred kits of the present invention comprise:

(i) the conjugate of Formula (Ia) or a salt thereof with a biocompatible counterion;
(ii) a radioprotectant which is chosen from ascorbic acid, *para*-aminobenzoic acid and, gentisic acid or biocompatible salts thereof;
(iii) a biocompatible reductant which comprises stannous.

Most preferred kits comprise ascorbic acid or a biocompatible salt thereof as the reductant. Stannous reductants are as described in the first embodiment (above).

When the conjugate of Formula (Ia) comprises an $N_2S_2$ diaminedithiol chelator, preferred kits of the present invention further comprise:

(i) the conjugate of Formula (Ia) where the tetradentate chelator comprises an $N_2S_2$ diaminedithiol of Formula II, or biocompatible salts thereof;
(ii) a radioprotectant which is chosen from ascorbic acid, *para*-aminobenzoic acid and, gentisic acid or biocompatible salts thereof;
(iii) a biocompatible reductant which comprises stannous;
(iv) a transchelator chosen from gluconic acid, glucoheptonic acid EDTA and biocompatible salts and combinations thereof.

When the $N_2S_2$ diaminedithiol chelator is that which forms part of TRODAT-1, the transchelator preferably comprises a combination of ethylenediaminetetraacetic acid (EDTA), and biocompatible salts thereof together with a salt of gluconic acid or glucoheptonic acid. When the tropane-tetradentate chelator conjugate is TRODAT-1, the kit of the present invention preferably comprises:

(i) the conjugate of Formula (IV) or a biocompatible salt thereof;
(ii) a radioprotectant which is chosen from ascorbic acid or biocompatible salts thereof;
(iii) a biocompatible reductant which comprises stannous;
(iv) a transchelator chosen from gluconic acid or glucoheptonic acid and biocompatible salts thereof;
(v) ethylenediaminetetraacetic acid (EDTA) and biocompatible salts thereof.

Preferred TRODAT-1 kits comprise ascorbic acid or sodium ascorbate as the radioprotectant and a combination of sodium gluconate and disodium edetate as the transchelator. A most preferred TRODAT-1 kit formulation is that given as Formulation P in Example 1.

In a fifth embodiment, the present invention provides a method of preparation of the radiopharmaceutical of the present invention, which comprises formation of the metal complex of the tropane chelator conjugate in a biocompatible carrier in a form suitable for mammalian administration by either:

(i) reaction of a radioactive isotope of technetium or rhenium with the precursor composition of the second embodiment in a biocompatible carrier in a form suitable for mammalian administration; or
(ii) forming the metal complex of the first embodiment in a biocompatible carrier in a form suitable for mammalian administration, and then subsequently adding an effective amount of at least one radioprotectant.

In a sixth embodiment, the present invention provides the use of the radiopharmaceutical of the third embodiment in a method of diagnostic imaging of the mammalian brain.

In a further embodiment, the present invention provides a method of diagnostic imaging of the mammalian brain which comprises imaging a mammal which had previously been administered with the radiopharmaceutical of the third embodiment. In this embodiment the radiopharmaceutical is used in a method of imaging, or image processing wherein the term "previously been administered" means that any step requiring a medically-qualified person to administer the agent to the patient has already been carried out.

The invention is illustrated by the following non-limiting Examples. Example 1 describes the materials and methods used in the comparative studies described in later Examples. Thus a formulation of the present invention (Formulation P) is compared with a prior art formulation (Formulation Q). Example 2 describes the radiolabelling protocol and quality control methodology employed.

Example 3 studies the effect of different $^{99m}$Tc generator elution characteristics on the performance of the kit formulations P and Q. $^{99m}$Tc generators are designed to be used over a period of several days, and depending on the age of the generator, and the time since the last elution, a range of characteristics of the eluate resulting from elution are obtained. A commercial kit must give satisfactory RCP preparations under the full range of storage and elution conditions in use by customers. Example 3 studies a range of four sets of elution conditions ("Elution Conditions 1 to 4). The results show that both formulations give acceptable initial RCPs (92 and 88% for P and Q respectively) under 'best case' generator elution conditions ("elution conditions 1"). Formulation P gave an RCP of 90% at 3.5 hours post-preparation, whereas for Formulation Q (prior art), the RCP fell off sharply to 77% over the same period of time. These results show that Formulation P exhibits both an improved initial RCP and an improved post-preparation stability.

Under generator elution conditions 2, Formulation P had an RCP of 92% immediately after preparation and 90% at 4 hours. The initial RCP for Formulation Q was 88%, but again it fell off significantly to 77% at 3.5 hours. These results demonstrate that even with generator eluate at the end of its usable shelf-life, Formulation P displays an improved initial RCP, and greater post-preparation stability than prior art Formulation Q.

Under generator elution conditions 3, in spite of the long interval between generator elutions, both formulations give acceptable initial RCPs (91 and 88% for P and Q respectively). At 4 hours post-preparation the RCP of Formulation P was still as high as 88%, whereas the RCP of Formulation Q was down to 73% after only 2 hours.

Under generator elution conditions 4, Formulation P exhibits an RCP of 90% after 1.5 hours and 89% at 3.5 hours post-preparation. When subjected to these most challenging eluate conditions, the RCP of prior art Formulation Q is only 76% at 1.5 hours, falling to 71% at 3.5 hours.

Example 4 shows that *p*-ABA is also effective as a radioprotectant for $^{99m}$Tc-TRODAT-1 preparations. Addition of *p*-ABA led to a significant improvement in RCP. Increasing the level of *p*-ABA from 200 to 500 $\mu$g increased further the stability at both initial and 4 hours.

Example 5 studies the effect of the volume of $^{99m}$Tc-pertechnetate used to reconstitute the kit vial ("reconstitution volume") on the RCP, at the same eluate radioactive concentration (0.75 GBq/ml). At the standard 2 ml reconstitution volume the Formulation P kits perform better than Formulation Q (prior art). The Formulation P kits continue to radiolabel well even when reconstituted with 3 GBq in 4 ml, but the RCP drops markedly when kits are reconstituted with 4.5 GBq in 6 ml. These results show that the RCP of both formulations are affected by reconstitution volume. This effect may be attributable to an increased path length effect for radiolysis of the solvent. The inclusion of the radioprotectant ascorbate in Formulation P suppresses the volume effect compared with the prior art Formulation (Q), but does not eliminate it completely.

Example 6 studies the effect of use of an autoclave heating cycle (121°C, 30 minutes) as part of the radiolabelling procedure, since Choi et al [Nucl.Med.Biol., 26, p. 461-466 (1999)] employ that vial heating methodology. The present experiments were typically conducted using heating *via* a boiling water bath, since the use of an autoclave as part of the preparation procedure is not an attractive option for a commercial product. Hence, a comparative study was carried out to determine if the different heating procedure might contribute to the RCP differences observed for Formulations

P and Q. Example 6 indicates that the use of an autoclave heating cycle has a detrimental effect on the RCP of both formulations. Low RCPs were observed for both formulations at both analysis time points and high levels of hydrophilic impurities were seen in the radioactive HPLC chromatograms. Hence, the reported stability of the prior art Choi *et al* $^{99m}$Tc-TRODAT-1 preparations cannot be ascribed to the use of autoclaving.

Example 7 shows that the useful regional brain biodistribution properties of $^{99m}$Tc-TRODAT-1 are maintained in the presence of the radioprotectant sodium ascorbate. Example 8 shows that a $^{99m}$Tc kit formulation of the present invention gives satisfactory RCP over a range of eluate conditions with three different commercial $^{99m}$Tc generators.

Example 9 shows that a Formulation P kit of the present invention can be reconstituted successfully at room temperature, using a two-step protocol. The radioactivity is added first, followed by a buffer solution at pH 7.4. The buffer raises the pH of the reaction mixture and drives the radiolabelling to completion.

Experimental.

A series of comparative experiments has been carried out to generate radiolabelling data for a radioprotectant formulation of the present invention vs the optimised one for $^{99m}$Tc-TRODAT-1 described in the prior art. The present formulation demonstrates significant advantages over the prior art published TRODAT-1 formulation [Choi et al, Nucl.Med.Biol., 26, p. 461-466 (1999)].

**Example 1: Materials and methods.**

All studies were carried out using lyophilised kit formulations. The kit vials were prepared under the same conditions but to different formulations - that of the present invention (Formulation P), and that of the prior art Choi *et al* formulation (Formulation Q). All vials were stored upright, in the dark at -20 ˚C until required for use. The $^{99m}$Tc-pertechnetate eluate was obtained from Amertec II™ generators (for Examples 3 to 7), Drytec™ generators (for Examples 8 and 9), and Ultra Technekow™ and Elutec™ generators (for Example 8). The kit formulations are given in Table 1:

**Table 1:** Present Kit Formulation (P) *vs* that of the Prior Art (Q).

| Kit components | Quantity of component per vial | |
|---|---|---|
| | Formulation P | Formulation Q (prior art) |
| TRODAT-1 | 10 μg* | 10 μg |
| $SnCl_2.2H_2O$ | 38 μg | 38 μg |
| Na-Glucoheptonate | 0 | 10 mg |
| Na-Gluconate | 10 mg | 0 |
| $Na_2EDTA.2H_2O$ | 840 μg | 840 μg |
| Na-Ascorbate | 500 μg | 0 |
| * Formulated as the trifluoroacetic acid salt | | |

The most significant difference is that Formulation P contains a radioprotectant (sodium ascorbate), whereas Formulation Q does not.

**Example 2: Radiolabelling procedure and purity determination.**

Unless otherwise stated, all test items were radiolabelled and analysed in the same way. Thus, once equilibrated to ambient temperature, each kit was reconstituted with 2 ml of sodium $^{99m}$Tc-pertechnetate solution containing 1.5 GBq (± 10 %) of radioactivity (1.5 GBq corresponds to 2 patient doses of 740 MBq), heated in a boiling water bath for 20 minutes and then cooled for 10 minutes before RCP analysis by HPLC and ITLC. Time of analysis is reported as 'post-preparation'.

RCP determination

HPLC:

Column: Xterra $RP_{18}$ 3.5 μm 3.0 x 50mm.
Loop size: 50μL,
Mobile Phase: 60% 50mM Ammonium Acetate pH 7: 40% Acetonitrile
Flow rate: 0.5ml/min.

ITLC:

Pall ITLC-SG sheet (part number 61886) cut into strips 20mm x 200mm and eluted with 50% 1M Ammonium Acetate : 50% Acetone
RCP calculation:
RCP = (A+B)*((100-RHT)/100)
A = species A from HPLC, B = species B from HPLC, RHT = reduced hydrolysed technetium, species at origin from ITLC.
Species A and Species B are the diastereomers of $^{99m}$Tc-TRODAT-1 as described by Meegalla et al [J.Med.Chem., 41, 428-436 (1998)].

**Example 3: Comparative kit performance for different generator elution conditions.**

Kits of formulations P and Q (as described in Example 1) were reconstituted, heated and analysed in exactly the same way, as per Example 2. Four generator elution conditions were investigated:

Generator elution conditions (1 to 4).

1. Fresh eluate: 24 hrs between elutions, <2hour old eluate;
2. Aged eluate: 24 hrs between elutions, >6 hour old eluate - high level of radiolysis products;
3. fresh eluate: 72 hrs between elutions, <2hour old eluate - low $^{99m}$Tc/$^{99}$Tc ratio);
4. aged eluate: 72 hrs between elutions, >6hour old eluate - low $^{99m}$Tc/$^{99}$Tc ratio and high level of radiolysis products.

RCP determinations were carried out at two post-preparation time points. The results are shown in Table 2:

**Table 2:** Radiolabelling of Formulations P and Q under four different generator elution conditions.

| Generator Elution Condition | Formulation | Time Post Preparation (h & min) | Mean %RCP (S.D.) | Mean A:B ratio |
|---|---|---|---|---|
| 1 | P (n = 3) | 0h 2 min<br>3h 27 min | 91.7 (1.2)<br>90.3 (1.1) | 45:55<br>47:53 |
| | Q (n = 3) | 0h 1 min<br>3h 28 min | 88.3 (1.9)<br>77.0 (1.5) | 46:54<br>56:44 |
| 2 | P (n = 3) | 0h 4 min<br>4h 2 min | 92.3 (1.2)<br>90.1 (1.0) | 45:55<br>50:50 |
| | Q (n = 3) | 0h 1 min<br>3h 59min | 88.0 (1.6)<br>75.1 (0.8) | 47:53<br>58:42 |
| 3 | P (n = 3) | 0h 1 min<br>4h 9 min | 91.0 (0.6)<br>88.3 (0.6) | 45:55<br>53:47 |
| | Q (n = 3) | 0h 2 min<br>2h 10 min | 87.2 (2.3)<br>73.0 (2.1) | 46:54<br>56:44 |

(continued)

| Generator Elution Condition | Formulation | Time Post Preparation (h & min) | Mean %RCP (S.D.) | Mean A:B ratio |
|---|---|---|---|---|
| 4 | P (n = 3) | 1h 40 min<br>3h 25 min | 89.8 (1.1)<br>88.7 (1.4) | 46:54<br>49:51 |
| | Q (n = 3) | 1h 40 min<br>3h 30 min | 76.2 (3.2)<br>70.5 (2.2) | 56:44<br>61:39 |

**Example 4: Effect of sodium *para*-aminobenzoate radioprotectant.**

A freshly prepared, nitrogen purged solution of sodium *p*-ABA (sodium para-aminobenzoate was added to a kit of Formulation Q. Radiolabelling of the kits was performed by first adding the radioprotectant (0.2 ml), followed by the immediate addition of pertechnetate solution (1 GBq in 1.8 ml). The kits were then heated as described in Example 2. The results are given in Table 3:

**Table 3:** Effect of addition of *p*-ABA radioprotectant.

| **Batch** | **Radioprotectant** | **% RCP (1 hour)** | **% RCP (4 hours)** |
|---|---|---|---|
| TRD009 | *p*-ABA (200 μg) | 90(n=2) | 86 (n=2) |
| TRD009 | None | 84 (n=2) | 76 (n=2) |
| | | | |
| TRD018 | *p*-ABA (200 μg) | 88 (n=2) | 84 (n=2) |
| TRD018 | None | 85 (n=2) | 77 (n=2) |
| | | | |
| TRD018 | *p*-ABA (500 μg) | 89 (n=2) | 87 (n=2) |
| TRD018 | None | 85 (n=2) | 78 (n=2) |

**Example 5: Effects of Reconstitution Volume.**

A comparison of the effects of reconstitution volume on the radiolabelling performance of the P and Q Formulations was carried out. Kits of both formulations were reconstituted, heated and analysed as per Examples 1 and 2. The radioactive concentration of eluate used to reconstitute the kits was kept constant at 1.5GBq/ml for each test item and eluate reconstitution volumes of 2, 4 and 6 ml were investigated.

**Table 4:** Effect of reconstitution volume on RCP of Formulations P and Q.

| Activity/Volume | Formulation | Time post-preparation (h & mins) | Mean %RCP (S.D.) | Mean A:B |
|---|---|---|---|---|
| 1.5 GBq / 2 ml | P (n=2) | 0h 4min<br>4h 6min | 91.4 (0.8)<br>88.7 (1.2) | 45:55<br>46:54 |
| | Q (n=2) | 0h 4min<br>4h 6min | 84.9 (0.4)<br>70.0 (2.8) | 48:52<br>58:42 |
| 3 GBq / 4 ml | P (n=2) | 0h 6min<br>4h 7min | 92.6 (1.5)<br>88.7 (0.4) | 45:55<br>50:50 |
| | Q (n=2) | 0h 5min<br>4h 9min | 81.7 (3.1)<br>60.7 (0.1) | 47:53<br>60:40 |

(continued)

| Activity/Volume | Formulation | Time post-preparation (h & mins) | Mean %RCP (S.D.) | Mean A:B |
|---|---|---|---|---|
| 4.5 GBq / 6 ml | P<br>(n=2) | 0h 4min<br>4h 7min | 77.2 (5.5)<br>74.0 (3.2) | 54:46<br>68:32 |
| | Q<br>(n=2) | 0h 4min<br>4h 5min | 70.6 (1.8)<br>41.6 (10.6) | 47:53<br>61:39 |

**Example 6: Study of the effect of heating using an autoclave.**

Two vials each of Formulations P and Q were reconstituted in the standard manner with 2 ml of sodium pertechnetate solution containing 1.5 GBq of radioactivity. The vials were subjected to an autoclave cycle of 121 ˚C for 25 minutes. The total duration of the cycle (heating and cooling) was about 120 minutes. As a result the earliest RCP analysis time point acquired was 2h 20 min post-reconstitution. Analysis times are reported in Table 5 as post-reconstitution (as opposed to post-preparation) time points:

**Table 5:** Comparative RCP of Formulations P and Q following an autoclave heating cycle (121 ˚C, 25 min).

| Formulation | Time Post Reconstitution (hr & min) | Mean %RCP | Mean Ratio A:B |
|---|---|---|---|
| P | 2h 35 min | 76.6 (n=2) | 50:50 |
| | 5h 43 min | 75.1 (n=2) | 55:45 |
| Q | 3h 03 min | 77.1 (n=2) | 54:46 |
| | 5h 40 min | 63.7 (n=2) | 60:40 |
| Control (P not autoclaved) | 0h 12 min | 89.1 (n=1) | 48:52 |

**Example 7: Study of the effect of an added radioprotectant on the biodistribution of $^{99m}$Tc-TRODAT-1.**

Kit formulation P was reconstituted to give $^{99m}$Tc-TRODAT-1 as described in Examples 1 and 2, which was the Test Item. The radiochemical purity (RCP) of the Test Item was 92% pre-injection, falling to 91% by the post-injection analysis time point. At the pre- and post-injection analysis time points, there were a low percentage of lipophilic (approximately 2%) and hydrophilic (approximately 6%) radiolabelled species present. The ratio of the A and diastereomers (46:54) remained constant at the pre- and post-injection analysis time points. Experiments were performed at 6 predetermined time points (2 and 20 minutes, 1, 2, 4 and 7 hours) post injection (p.i.) of the Test Item in normal male Wistar rats (180 to 220 g). Animals were anaesthetised with Halothane (6 % in oxygen), injected with 0.1 ml (500 MBq/ml) Test Item, sacrificed, dissected and the samples assayed for radioactivity. A comparative study was carried out using a $^{99m}$Tc-TRODAT-1 kit preparation corresponding to Formulation P, but lacking the ascorbate radioprotectant.

The percentage of the injected dose present in the blood was approximately three-fold lower for Formulation P at all the time points post-injection. The uptake and retention of radioactivity into the brain was similar at all except the 20 minute pi time point for both formulations. By 20 minutes pi, approximately 0.45% of the injected dose (id) was retained within the brain after administration of the radioprotectant formulation, relative to 0.29 % id after administration for the unstabilised formulation. This difference in brain uptake was reflected in the elevated percentage injected dose present in the brain regions at 20 minutes pi when expressed per gram of brain region.

The main difference observed was the elevated selective retention in the striatum after administration of the radioprotectant formulation, which peaked at 2.31 ± 0.31 after 2 hours pi and stayed at this peak level out to 4 hours pi (2.42 ± 0.80). In comparison, after administration of the unstabilised formulation the selective retention in the striatum was 1.74 ± 0.96 by 2 hours pi and 0.76 ± 0.30 by 4 hours pi.

**Example 8: Compatibility Study of a Kit Formulation of the Present Invention with Commercial $^{99m}$Tc Generators.**

Kits of Formulation P of the present invention were reconstituted with 2 GBq of $^{99m}$Tc in 2.5 ml of eluate from 3 different European $^{99m}$Tc generators, heated and cooled as per Example 2 and then stored at either 5 ˚C or 25 ˚C and analysed at 0, 4 and 6 hours post-preparation. Tests were carried out on kits reconstituted with both fresh and aged eluate from $^{99m}$Tc-generators. The results are shown in Table 7 (overleaf):

**Example 9: Alternative Room Temperature Reconstitution Conditions for a Kit Formulation of the Present Invention.**

The kit of Formulation P was reconstituted in two steps. First 1.5 ml of $^{99m}$Tc sodium pertechnetate solution containing 2GBq of radioactivity was added to the kit vial. Phosphate buffer solution of pH 7.4 (1 ml) was then added immediately, and the RCP determined 30 minutes after the addition of the pertechnetate solution. The results are given in Table 6:

**Table 6:** RCP of a kit reconstituted *via* a 2-step room temperature protocol.

| Preparation # | Mean %RCP | Mean A:B |
|---|---|---|
| 1 | 86.4 | 47:53 |
| 2 | 85.7 | 47:53 |
| 3 | 87.4 | 44:56 |
| 4 | 92.0 | 51:49 |

**Table 7:** RCP data for Formulation P kits reconstituted with fresh and aged eluate from 3 European $^{99m}$Tc-generators.

| Analysis time point (hours) | %RCP at 5˚C | %RCP at 25˚C |
|---|---|---|
| **1. Drytec™ (Amersham Health).** | | |
| Fresh eluate (24hr between elutions and <2 hours old) | | |
| 0 | 94 | 94 |
| 4 | 91 | 92 |
| 6 | 90 | 90 |
| Old eluate (72hr between elutions and >6 hours old) | | |
| 0 | 92 | 92 |
| 4 | 89 | 89 |
| 6 | 91 | 88 |
| | | |
| **2. Ultra Technekow™ (Tyco/Mallinckrodt).** | | |
| Fresh eluate (24hr between elutions and <2 hours old) | | |
| 0 | 94 | 95 |
| 4 | 93 | |
| 6 | 92 | 92 |
| Old eluate (72hr between elutions and >6 hours old) | | |
| 0 | 92 | 94 |
| 4 | 90 | 92 |
| 6 | 89 | 90 |
| | | |
| **3. Elutec™ (SMS/Nordion).** | | |
| Old eluate (72hr between elutions and >6 hours old) | | |
| 0 | 94 | 94 |
| 4 | 91 | 92 |
| 6 | 92 | 90 |

(continued)

| Analysis time point (hours) | %RCP at 5˚C | %RCP at 25˚C |
|---|---|---|
| Old eluate (72hr between elutions and >6 hours old) | | |
| 0 | 92 | 90 |
| 4 | 91 | 87 |
| 6 | 90 | 86 |

## Claims

**1.** A stabilised composition which comprises:

(i) a metal complex of a radioactive isotope of technetium or rhenium chelated to a conjugate, wherein said conjugate comprises a tetradentate chelating agent conjugated to a tropane, and said tetradentate chelating agent forms a neutral metal complex with said radioactive isotope of technetium or rhenium;
(ii) at least one radioprotectant.

**2.** The stabilised composition of claim 1, wherein the conjugate is of Formula Ia:

$$[\{\text{tropane}\}\text{-}(A)_n]_m\text{-}[\text{tetradentate chelating agent}] \quad \text{(Ia)}$$

where:

$-(A)_n-$ is a linker group wherein each A is independently $-CR_2-$, $-CR{=}CR-$, $-C{\equiv}C-$ , $-CR_2CO_2-$ , $-CO_2CR_2-$ , $-NRCO-$, $-CONR-$, $-NR(C{=}O)NR-$, $-NR(C{=}S)NR-$, $-SO_2NR-$ , $-NRSO_2-$ , $-CR_2OCR_2-$ , $-CR_2SCR_2-$ , $-CR_2NRCK_2-$ , a $C_{4-8}$ cycloheteroalkylene group, a $C_{4-8}$ cycloalkylene group, a $C_{5-12}$ arylene group, or a $C_{3-12}$ heteroarylene group;
R is independently chosen from H, $C_{1-4}$ alkyl, $C_{2-4}$ alkenyl, $C_{2-4}$ alkynyl, $C_{1-4}$ alkoxyalkyl or $C_{1-4}$ hydroxyalkyl;
n is an integer of value 0 to 10; and,
m is 1, 2 our 3.

**3.** The stabilised composition of claims 1 or 2, where the tropane is a phenyl tropane of Formula III:

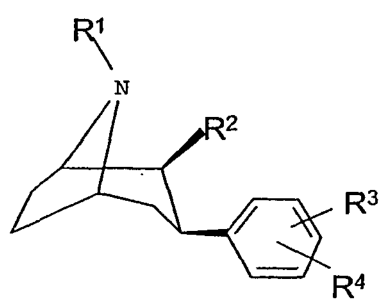

(III)

where:

$R^1$ is H, $C_{1-4}$ alkyl, $C_{1-4}$ alkenyl or $C_{1-4}$ fluoroalkyl;
$R^2$ is $CO_2R^5$, $CON(R^5)_2$, $COR^5$ or $C_{1-4}$ alkyl, where each $R^5$ is independently H or $C_{1-3}$ alkyl;
$R^3$ and $R^4$ are independently H, Cl, Br, F, I, $CH_3$, $CF_3$, $NO_2$, $OCH_3$ or $NH_2$.

**4.** The stabilised composition of claims 1 to 3, wherein the radioactive isotope of technetium is $^{99m}Tc$.

**5.** The stabilised composition of claims 1 to 4, wherein the tetradentate chelating agent is conjugated to either the 2- or the 8- position of the tropane.

**6.** The stabilised composition of claim 5, wherein the tetradentate chelating agent is conjugated to the 2- position of the tropane.

**7.** The stabilised composition of claims 1 to 6, wherein the tetradentate chelating agent has a donor set chosen from:

(i) $N_2S_2$;
(ii) $N_3S$;
(iii) diaminedioxime, or
(iv) amidetriamine.

**8.** The stabilised composition of claims 1 to 7, wherein the tetradentate chelating agent has an $N_2S_2$ diaminedithiol donor set.

**9.** The stabilised composition of claims 1 to 8, wherein the radioprotectant comprises ascorbic acid, *para*-aminobenzoic acid or gentisic acid, or a salt thereof with a biocompatible cation.

**10.** The stabilised composition of claim 9, wherein the radioprotectant comprises ascorbic acid or a salt thereof with a biocompatible cation.

**11.** The stabilised composition of claim 10, wherein the radioprotectant comprises ascorbic acid or sodium ascorbate.

**12.** The stabilised composition of claims 1 to 11 wherein the metal complex comprises the radioactive technetium isotope $^{99m}Tc$;
the tetradentate chelating agent has an $N_2S_2$ diaminedithiol donor set and is conjugated to the 2-position of a phenyl tropane of Formula III of Claim 3; and the radioprotectant comprises ascorbic acid or a salt thereof with a biocompatible cation.

**13.** The stabilised composition of claim 12, wherein the metal complex has the Formula:

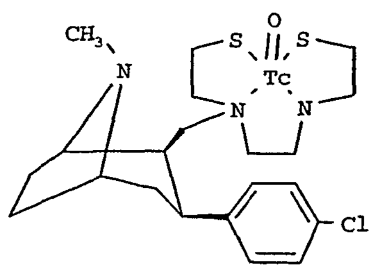

**14.** A precursor composition useful in the preparation of the stabilised composition of claims 1 to 13, said precursor composition comprising:

(i) the conjugate of Formula Ia as defined in claim 2;
(ii) the radioprotectant of claim 1 and claims 9 to 11.

**15.** The precursor composition of claim 14, where the tropane of the conjugate of Formula Ia is a phenyl tropane of Formula III of claim 3.

**16.** The precursor composition of claims 14 or 15 where the chelator conjugate is of Formula IV:

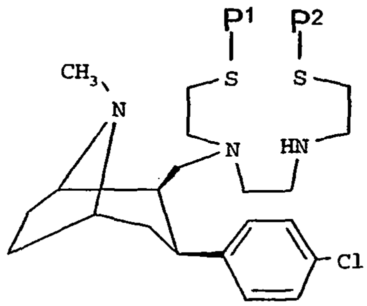

(IV)

where $P^1$ and $P^2$ are independently H or a thiol protecting group.

**17.** The precursor composition of claim 16, where $P^1$ and $P^2$ are both H.

**18.** A radiopharmaceutical which comprises the stabilised composition of claims 1-13 together with a biocompatible carrier, in a form suitable for mammalian administration.

**19.** The radiopharmaceutical of claim 18, where the radiopharmaceutical is provided in a syringe.

**20.** The radiopharmaceutical of claim 18, where the radiopharmaceutical is provided in a vial fitted with a closure, wherein said closure is suitable for maintaining sterile integrity when punctured with a needle.

**21.** A kit for the preparation of the radiopharmaceutical of claims 18 to 20, which comprises:

(i) the conjugate of Formula (Ia) of claim 2 or a salt of said conjugate with a biocompatible counterion;
(ii) the radioprotectant of claim 1;
(iii) a biocompatible reductant.

**22.** The kit of claim 21, where the radioprotectant is as defined in claims 9 to 11.

**23.** The kit of claims 21 or 22, where the biocompatible reductant comprises stannous.

**24.** A method of preparation of the radiopharmaceutical of claims 18 - 20, which comprises formation of the metal complex of claims 1 to 8 in a biocompatible carrier in a form suitable for mammalian administration by either:

(i) reaction of a radioactive isotope of technetium or rhenium with the precursor composition of claims 14 to 17 in a biocompatible-carrier in a form suitable for mammalian administration; or
(ii) forming the metal complex of claims 1 to 8 in a biocompatible carrier in a form suitable for mammalian administration, and then subsequently adding an effective amount of at least one radioprotectant.

**25.** The method of claim 24, where the metal complex comprises the conjugate of Formula Ia of claim 2.

**26.** The method of claims 24 or 25, where the radioprotectant is as defined in claims 9 to 11.

**27.** Use of the radiopharmaceutical of claims 18-20 in a method of diagnostic imaging of the mammalian body.

**28.** A method of diagnostic imaging of the mammalian body which comprises imaging a mammal which had previously been administered with the radiopharmaceutical of claims 18-20.

**Patentansprüche**

**1.** Stabilisierte Zusammensetzung, die umfasst:

(i) einen Metallkomplex aus einem radioaktiven Technetium- oder Rheniumisotop, das an ein Konjugat chelatisiert ist, wobei das Konjugat einen dreizähnigen Chelatbildner umfasst, der an ein Tropan konjugiert ist, und der dreizähnige Chelatbildner einen neutralen Metallkomplex mit dem radioaktiven Technetium- oder Rheniumisotop bildet;
(ii) zumindest ein Strahlenschutzmittel bzw. einen Radioprotektor.

**2.** Stabilisierte Zusammensetzung nach Anspruch 1, wobei das Konjugat der Formel Ia entspricht:

$$[\{Tropan\}-(A)_n]_m-[dreizähniger\ Chelatbildner] \quad (Ia)$$

worin:

$-(A)_n-$ für eine Linkergruppe steht, wobei jedes A unabhängig steht für $-CR_2-$, $-CR=CR-$, $-C\equiv C-$, $-CR_2CO_2-$, $-CO_2CR_2-$, $-NRCO-$, $-CONR-$, $-NR(C=O)NR-$, $-NR(C=S)NR-$, $-SO_2NR-$, $-NRSO_2-$, $-CR_2OCR_2-$, $-CR_2SCR_2-$, $-CR_2NRCR_2-$, eine $C_{4-8}$-Cycloheteroalkylengruppe, eine $C_{4-8}$-Cycloalkylengruppe, eine $C_{5-12}$-Arylengruppe oder eine $C_{3-12}$-Heteroarylengruppe;
R unabhängig gewählt ist aus H, $C_{1-4}$-Alkyl, $C_{2-4}$-Alkenyl, $C_{2-4}$-Alkinyl, $C_{1-4}$-Alkoxyalkyl oder $C_{1-4}$-Hydroxyalkyl;
n für eine ganze Zahl vom Wert 0 bis 10 steht; und
m für 1, 2 oder 3 steht.

**3.** Stabilisierte Zusammensetzung nach Anspruch 1 oder 2, wobei das Tropan ein Phenyltropan der Formell III ist:

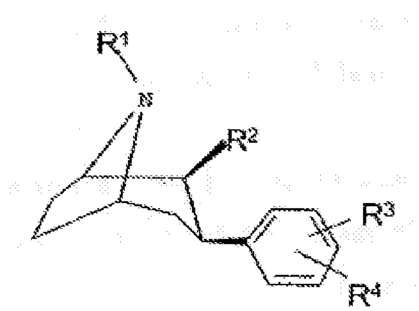

(III)

worin:

$R^1$ für H, $C_{1-4}$-Alkyl, $C_{1-4}$-Alkenyl oder $C_{1-4}$-Fluoralkyl steht;
$R^2$ für $CO_2R^5$, $CON(R^5)_2$, $COR^5$ oder $C_{1-4}$-Alkyl steht, wobei jedes $R^5$ unabhängig für H oder $C_{1-3}$-Alkyl steht;
$R^3$ und $R^4$ unabhängig für H, Cl, Br, F, I, $CH_3$, $CF_3$, $NO_2$, $OCH_3$ oder $NH_2$ stehen.

**4.** Stabilisierte Zusammensetzung nach Anspruch 1 bis 3, wobei das radioaktive Technetiumisotop $^{99m}Tc$ ist.

**5.** Stabilisierte Zusammensetzung nach Anspruch 1 bis 4, wobei der dreizähnige Chelatbildner an entweder die 2- oder die 8-Position des Tropans konjugiert ist.

**6.** Stabilisierte Zusammensetzung nach Anspruch 5, wobei der dreizähnige Chelatbildner an die 2-Position des Tropans konjugiert ist.

**7.** Stabilisierte Zusammensetzung nach Anspruch 1 bis 6, wobei der dreizähnige Chelatbildner einen Donorsatz aufweist, gewählt aus:

(i) $N_2S_2$;
(ii) $N_3S$;
(iii) Diamindioxim, oder

(iv) Amidtriamin.

**8.** Stabilisierte Zusammensetzung nach Anspruch 1 bis 7, wobei der dreizähnige Chelatbildner einen $N_2S_2$-Diamindithiol-Donorsatz aufweist.

**9.** Stabilisierte Zusammensetzung nach Anspruch 1 bis 8, wobei das Strahlenschutzmittel bzw. der Radioprotektor Ascorbinsäure, *para*-Aminobenzoesäure oder Gentisinsäure, oder ein Salz davon mit einem biokompatiblen Kation, umfasst.

**10.** Stabilisierte Zusammensetzung nach Anspruch 9, wobei das Strahlenschutzmittel bzw. der Radioprotektor Ascorbinsäure oder ein Salz davon mit einem biokompatiblen Kation umfasst.

**11.** Stabilisierte Zusammensetzung nach Anspruch 10, wobei das Strahlenschutzmittel bzw. der Radioprotektor Ascorbinsäure oder Natriumascorbat umfasst.

**12.** Stabilisierte Zusammensetzung nach Anspruch 1 bis 11, wobei der Metallkomplex das radioaktive Technetiumisotop $^{99m}$Tc umfasst;
der dreizähnige Chelatbildner einen $N_2S_2$-Diamindithiol-Donorsatz aufweist und an die 2-Position eines Phenyltropans der Formel III des Anspruchs 3 konjugiert ist;
und das Strahlenschutzmittel bzw. der Radioprotektor Ascorbinsäure oder ein Salz davon mit einem biokompatiblen Kation umfasst.

**13.** Stabilisierte Zusammensetzung nach Anspruch 12, wobei der Metallkomplex der Formel entspricht:

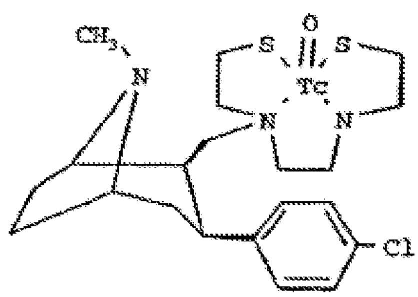

**14.** Präkursorzusammensetzung, verwendbar bei der Herstellung der stabilisierten Zusammensetzung der Ansprüche 1 bis 13, wobei die Präkursorzusammensetzung umfasst:

(i) das Konjugat der Formel Ia wie in Anspruch 2 definiert;
(ii) das Strahlenschutzmittel bzw. den Radioprotektor des Anspruchs 1 und der Ansprüche 9 bis 11.

**15.** Präkursorzusammensetzung nach Anspruch 14, wobei das Tropan des Konjugats der Formel Ia ein Phenyltropan der Formel III des Anspruchs 3 ist.

**16.** Präkursorzusammensetzung nach Anspruch 14 oder 15, wobei das Chelatorkonjugat der Formel IV entspricht:

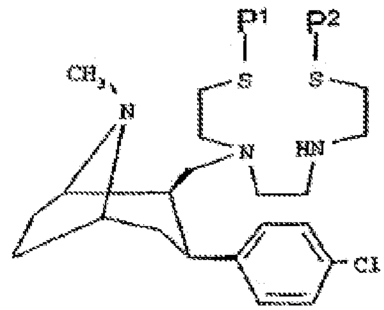

(IV)

worin $P^1$ und $P^2$ unabhängig für H oder eine Thiolschutzgruppe stehen.

**17.** Präkursorzusammensetzung nach Anspruch 16, wobei $P^1$ und $P^2$ beide für H stehen.

**18.** Radiopharmakon, das die stabilisierte Zusammensetzung der Ansprüche 1 bis 13 zusammen mit einem biokompatiblen Träger, in einer zur Verabreichung an Säuger geeigneten Form, umfasst.

**19.** Radiopharmakon nach Anspruch 18, wobei das Radiopharmakon in einer Spritze bereitgestellt wird.

**20.** Radiopharmakon nach Anspruch 18, wobei das Radiopharmakon in einem mit Verschluss versehenen Fläschchen bereitgestellt wird, wobei der Verschluss geeignet ist, um die sterile Integrität aufrechtzuerhalten, wenn er mit einer Nadel durchstochen wird.

**21.** Kit zur Herstellung des Radiopharmakons der Ansprüche 18 bis 20, das umfasst:

(i) das Konjugat der Formel Ia des Anspruchs 2 oder ein Salz des Konjugats mit einem biokompatiblen Gegenion;
(ii) das Strahlenschutzmittel bzw. den Radioprotektor des Anspruchs 1;
(iii) ein biokompatibles Reduktionsmittel.

**22.** Kit nach Anspruch 21, wobei das Strahlenschutzmittel bzw. der Radioprotektor wie in Ansprüchen 9 bis 11 definiert ist.

**23.** Kit nach Anspruch 21 oder 22, wobei das biokompatible Reduktionsmittel Zinn umfasst.

**24.** Verfahren zur Herstellung des Radiopharmakons der Ansprüche 18 bis 20, das die Bildung des Metallkomplexes der Ansprüche 1 bis 8 in einem biokompatiblen Träger in einer zur Verabreichung an Säuger geeigneten Form umfasst durch entweder:

(i) Umsetzung eines radioaktiven Technetium- oder Rheniumisotops mit der Präkursorzusammensetzung der Ansprüche 14 bis 17 in einem biokompatiblen Träger in einer zur Verabreichung an Säuger geeigneten Form; oder
(ii) Bildung des Metallkomplexes der Ansprüche 1 bis 8 in einem biokompatiblen Träger in einer zur Verabreichung an Säuger geeigneten Form, und anschließendes Hinzugeben einer wirksamen Menge von zumindest einem Strahlenschutzmittel bzw. Radioprotektor.

**25.** Verfahren nach Anspruch 24, wobei der Metallkomplex das Konjugat der Formel Ia des Anspruchs 2 umfasst.

**26.** Verfahren nach Anspruch 24 oder 25, wobei das Strahlenschutzmittel bzw. der Radioprotektor wie in Ansprüchen 9 bis 11 definiert ist.

**27.** Verwendung des Radiopharmakons der Ansprüche 18 bis 20 in einem Verfahren zur diagnostischen Bildgebung vom Säugerkörper.

**28.** Verfahren zur diagnostischen Bildgebung vom Säugerkörper, das das Abbilden eines Säugers umfasst, dem zuvor

das Radiopharmakon der Ansprüche 18 bis 20 verabreicht worden ist.

**Revendications**

1. Composition stabilisée qui comprend :

   (i) un complexe métallique d'un isotope radioactif de technétium ou de rhénium chélaté à un conjugué, dans laquelle ledit conjugué comprend un agent de chélation tétradentate conjugué à un tropane, et ledit agent de chélation tétradentate forme un complexe métallique neutre avec ledit isotope radioactif de technétium ou de rhénium ;
   (ii) au moins un agent radioprotecteur.

2. Composition stabilisée selon la revendication 1, dans laquelle le conjugué est suivant la formule la :

$$[\{tropane\}-(A)_n]_m-[\text{ agent de chélation tétradentate}] \quad (Ia)$$

   dans laquelle :

   $-(A)_n-$ est un groupe lieur dans lequel chaque A représente, de manière indépendante, $-CR_2-$, -CR=CR-, -C≡C-, $-CR_2CO_2-$, $-CO_2CR_2-$, -NRCO-, - CONR-, -NR(C=C)NR-, -NR(C=S)NR-, $-SO_2NR-$, $-NRSO_2-$, - $CR_2OCR_2-$, - $CR_2SCR_2-$, $-CR_2NRCR_2-$, un groupe cyclohétéro-alkylène en $C_{4-8}$, un groupe cyclo-alkylène en $C_{4-8}$, un groupe arylène en $C_{5-12}$, ou un groupe hétéro-arylène en $C_{3-12}$ ;
   R est, de manière indépendante, choisi à partir de H, un alkyle en $C_{1-4}$, un alkényle en $C_{2-4}$, un alkynyle en $C_{2-4}$, un alkoxy-alkyle en $C_{1-4}$ ou un hydroxy-alkyle en $C_{1-4}$ ;
   n est un entier de valeur comprise entre 0 et 10 ; et,
   m est égal à 1, 2 ou 3.

3. Composition stabilisée selon les revendications 1 ou 2, dans laquelle le tropane est un phényle tropane suivant la formule III :

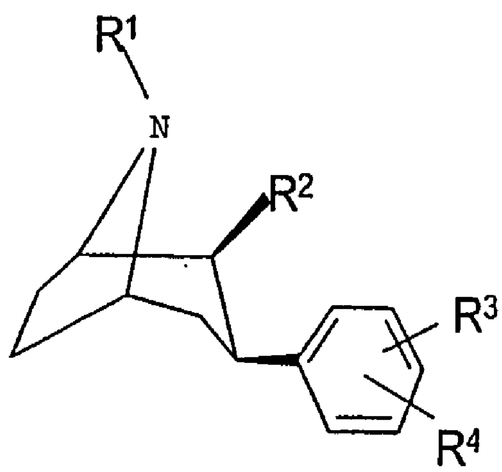

(III)

   dans laquelle :

   $R_1$ représente H, un alkyle en $C_{1-4}$, un alkényle en $C_{1-4}$ ou un fluoroalkyle en $C_{1-4}$ ;
   $R_2$ représente $CO_2R_5$, $CON(R_5)_2$, $COR_5$ ou un alkyle en $C_{1-4}$, dans lesquels chaque $R_5$ représente, de manière

indépendante, H ou un alkyle en $C_{1-3}$ ;
$R_3$ et $R_4$ représentent, de manière indépendante, H, Cl, Br, F, I, $CH_3$, $CF_3$, $NO_2$, $OCH_3$ ou $NH_2$

**4.** Composition stabilisée selon les revendications 1 à 3, dans laquelle l'isotope radioactif de technétium est le $^{99m}$Tc.

**5.** Composition stabilisée selon les revendications 1 à 4, dans laquelle l'agent de chélation tétradentate est conjugué à la position soit 2, soit 8 du tropane.

**6.** Composition stabilisée selon la revendication 5, dans laquelle l'agent de chélation tétradentate est conjugué à la position 2 du tropane.

**7.** Composition stabilisée selon les revendications 1 à 6, dans laquelle l'agent de chélation tétradentate comporte un ensemble donneur choisi à partir de :

(i) $N_2S_2$ ;
(ii) $N_3S$ ;
(iii) diaminedioxime, ou
(iv) amidetriamine.

**8.** Composition stabilisée selon les revendications 1 à 7, dans laquelle l'agent de chélation tétradentate comporte un ensemble donneur à base de $N_2S_2$ diaminedithiol.

**9.** Composition stabilisée selon les revendications 1 à 8, dans laquelle l'agent radioprotecteur comprend de l'acide ascorbique, de l'acide para-aminobenzoïque ou de l'acide gentisique, ou un sel de ceux-ci avec un cation biocompatible.

**10.** Composition stabilisée selon la revendication 9, dans laquelle l'agent radioprotecteur comprend de l'acide ascorbique ou un sel de celui-ci avec un cation biocompatible.

**11.** Composition stabilisée selon la revendication 10, dans laquelle l'agent radioprotecteur comprend de l'acide ascorbique ou de l'ascorbate de sodium.

**12.** Composition stabilisée selon les revendications 1 à 11, dans laquelle le complexe métallique comprend l'isotope radioactif de technétium $^{99m}$Tc ;
l'agent de chélation tétradentate comporte un ensemble donneur $N_2S_2$ diaminedithiol et est conjugué à la position 2 d'un phényle tropane de formule III selon la revendication 3 ;
et l'agent radioprotecteur comprend de l'acide ascorbique ou un sel de celui-ci avec un cation biocompatible.

**13.** Composition stabilisée selon la revendication 12, dans laquelle le complexe métallique présente la formule :

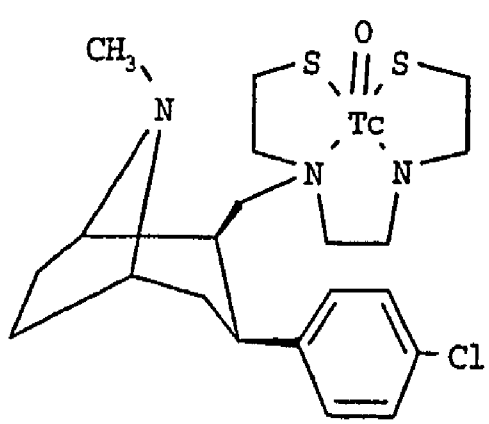

**14.** Composition de précurseur pouvant être utilisée dans la préparation de la composition stabilisée selon les revendications 1 à 13, ladite composition de précurseur comprenant :

(i) le conjugué suivant la formule la selon la revendication 2 ;
(ii) le radioprotecteur selon la revendication 1 et les revendications 9 à 11.

**15.** Composition de précurseur selon la revendication 14, dans laquelle le tropane du conjugué de formule la est un tropane phényle de formule III selon la revendication 3.

**16.** Composition de précurseur selon les revendications 14 ou 15, dans laquelle le conjugué chélateur est suivant la formule IV :

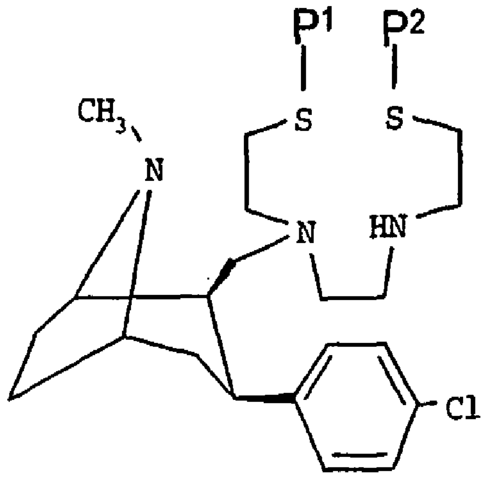

(IV)

dans laquelle $P^1$ et $P^2$ sont indépendamment H ou un groupe protecteur thiol.

**17.** Composition de précurseur selon la revendication 16, dans laquelle $P^1$ et $P^2$ représentent tous les deux H.

**18.** Agent radio-pharmaceutique qui comprend la composition stabilisée selon les revendications 1 à 13, ensemble avec un support biocompatible, sous une forme appropriée à l'administration à un mammifère.

**19.** Agent radio-pharmaceutique selon la revendication 18, dans lequel l'agent radio-pharmaceutique est délivré dans une seringue.

**20.** Agent radio-pharmaceutique selon la revendication 18, dans lequel l'agent radio-pharmaceutique est délivré dans une éprouvette comportant un obturateur, dans lequel ledit obturateur est approprié afin d'assurer le maintien de l'intégrité stérile lorsqu'il est perforé avec une aiguille.

**21.** Kit de préparation de l'agent radio-pharmaceutique selon les revendications 18 à 20, qui comprend :

(i) le conjugué de formule (la) selon la revendication 2 ou un sel dudit conjugué avec un contre-ion biocompatible ;
(ii) l'agent radioprotecteur selon la revendication 1 ;
(iii) un agent réducteur biocompatible.

**22.** Kit selon la revendication 21, dans lequel l'agent radioprotecteur est selon les revendications 9 à 11.

**23.** Kit selon les revendications 21 ou 22, dans lequel l'agent réducteur biocompatible comprend un élément stanneux.

**24.** Procédé de préparation du produit radio-pharmaceutique selon les revendications 18 à 20, qui comprend la formation du complexe métallique suivant les revendications 1 à 8 sur un support biocompatible sous une forme appropriée à l'administration à un mammifère soit :

(i) par réaction d'un isotope radioactif de technétium ou de rhénium avec la composition de précurseur selon les revendications 14 à 17 sur un support biocompatible sous une forme appropriée à l'administration à un mammifère ; soit

(ii) par formation du complexe métallique suivant les revendications 1 à 8 sur un porteur biocompatible sous une forme appropriée à l'administration à un mammifère, et ensuite, par ajout consécutif d'une quantité efficace d'au moins un agent radioprotecteur.

**25.** Procédé selon la revendication 24, dans lequel le complexe métallique comprend le conjugué de formule la selon la revendication 2.

**26.** Procédé selon les revendications 24 ou 25, dans lequel l'agent radioprotecteur est selon les revendications 9 à 11.

**27.** Utilisation de l'agent radio-pharmaceutique selon les revendications 18 à 20 dans un procédé d'imagerie diagnostique du corps mammifère.

**28.** Procédé d'imagerie diagnostique du corps mammifère qui comprend l'imagerie d'un mammifère auquel a préalablement été administré l'agent radio-pharmaceutique selon les revendications 18 à 20.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5980860 A
- US 6171576 B
- WO 03055879 A
- WO 02053192 A
- WO 9855154 A
- EP 0078642 A
- EP 0004684 A
- GB 1489330 A


### Non-patent literature cited in the description


- **Morgan ; Nowotnik.** *Drug News Perspect,* 1999, vol. 12 (3), 137-145
- **Kung.** *Nucl.Med.Biol.,* 2001, vol. 28, 505-508
- **Meltzer et al.** *J.Med.Chem.,* 1997, vol. 40, 1835-1844
- **Meegalla et al.** *J.Med.Chem.,* 1997, vol. 40, 9-17
- **Fan et al.** *Chin.J.Nucl.Med.,* 1999, vol. 19 (3), 146-148
- **Choi et al.** *Nucl.Med.Biol.,* 1999, vol. 26, 461-466
- Radiopharmaceuticals and Methods of Radiolabeling. **Saha, G. B.** Fundamentals of Nuclear Pharmacy. 80-108
- **Hung et al.** *Cardiolite™ [Nucl. Med. Biol.,* 1996, vol. 23, 599-603
- **Jurisson et al.** *Chem.Rev.,* 1999, vol. 99, 2205-2218
- **Arano et al.** *Chem.Pharm.Bull.,* 1991, vol. 39, 104-107
- **McBride et al.** *J.Med.Chem.,* 1993, vol. 36, 81-6
- **Turpin et al.** *J.Lab.Comp.Radiopharm.,* 2002, vol. 45, 379-393
- **Nanjappan et al.** *Tetrahedron,* 1994, vol. 50, 8617-8632
- **Bryson et al.** *Inorg.Chem.,* 1990, vol. 29, 2948-2951
- **Pillai et al.** *Appl.Rad.Isot.,* 1990, vol. 41, 557-561
- **Volkert et al.** *Radiochim. Acta,* 1993, vol. 63, 205-208
- **Theorodora W. Greene ; Peter G. M. Wuts.** Protective Groups in Organic Synthesis. John Wiley & Sons, 1991
- **Arano.** *Adv. Drug Deliv. Rev.,* 1999, vol. 37, 103-120
- **Meltzer et al.** N2S2 amideaminedithiol (MAMA) chelators. *J. MED. CHEM.,* 1997, vol. 40, 1835-1844
- **Meegalla et al.** *J.Med.Chem.,* 1998, vol. 41, 428-436